# EUROPEAN PATENT APPLICATION

(11) **EP 4 563 587 A1**
(43) Date of publication of application: **04.06.2025**
(21) Application number: 23846863.1
(22) Date of filing: 10.07.2023
(51) Int. Cl.: C07K 14/00, A01N 63/50, A61K 38/00, A61P 31/04, C12N 15/70, C12R 1/19

(54) **ANTIBACTERIAL PROTEIN HAVING STRONG LYTIC ACTIVITY TO STAPHYLOCOCCI INCLUDING STAPHYLOCOCCUS AUREUS**

(30) Priority: 26.07.2022 KR 20220092185
(71) Applicant: Intron Biotechnology, Inc., Seongnam-si, Gyeonggi-do 13202 (KR)
(72) Inventor: YOON, Seong Jun, Seoul 06281 (KR); JUN, Soo Youn, Seoul 06518 (KR); JUNG, Gi Mo, Seoul 08725 (KR); CHOI, Ji Hye, Seongnam-si Gyeonggi-do 13245 (KR); KANG, Sang Hyeon, Seoul 05501 (KR)
(74) Representative: Latscha Schöllhorn Partner AG
(86) International application number: PCT/KR2023/009721
(87) International publication number: WO 2024/025204

(57) **Abstract**

The present invention relates to antibacterial protein S2200 which has specific antibacterial activity against Staphylococci including *Staphylococcus aureus. More specifically, the present invention relates to: antibacterial protein S2200 specific for Staphylococci including Staphylococcus aureus, which has the ability to specifically lyse Staphylococci including Staphylococcus aureus and has the amino acid sequence represented by SEQ ID NO: 1; and a pharmaceutical composition for the treatment of infections caused by Staphylococci including Staphylococcus aureus, containing as an active ingredient antibacterial protein S2200 specific for Staphylococci including Staphylococcus aureus.*

## Description

### Technical Field

The present disclosure relates to an antibacterial protein S2200, which has lytic activity against *Staphylococci,* including *Staphylococcus aureus.* More specifically, the present disclosure relates to an antibacterial protein S2200 specific for the *Staphylococci,* the antibacterial protein S2200 having an ability to specifically lyse *staphylococci,* which can cause infectious diseases, and containing an amino acid sequence represented by SEQ ID NO: 1. The present disclosure also relates to a pharmaceutical composition for treating infectious diseases caused by *Staphylococci,* containing the staphylococci-specific antibacterial protein S2200 as an active ingredient.

### Background Art

*Staphylococcus aureus* was first discovered in a sample of purulent inflammation by German scientist Robert Koch in 1878 and was given the genus name *Staphylococcus* by Alexander Ogston of Scotland in 1881. *Staphylococcus aureus* is a facultatively anaerobic gram-positive coccus and is the most common cause of staphylococcal infections.

*Staphylococcus aureus* is one of the pathogens that cause infections in humans in hospitals and the community, so *Staphylococcus aureus* is the causative agent of fatal diseases such as pneumonia, endocarditis, and sepsis. In particular, infectious diseases (*Staphylococcus aureus* infections of 50% or more and mortality rate of approximately 15%) caused by methicillin-resistant *S. aureus* (MRSA) pose a serious risk to human health.

To treat these *Staphylococcus aureus* infections, antibiotics have been widely used. Recently, as *Staphylococcus aureus* continues to acquire resistance to antibiotics, there is a problem that effectiveness of antibiotic treatment is seriously reduced. Therefore, there is a need to develop new antibacterial substances effective in treating infections caused by *Staphylococcus aureus,* which has acquired resistance to conventional antibiotics. In particular, there is a great need for the development of pharmaceutical agents, which can quickly demonstrate antibacterial activity and have new mechanisms of action.

### Disclosure

### Technical Problem

Accordingly, as a solution to the problems caused by the use of the conventional antibiotics against the harmful pathogenic bacteria *Staphylococci,* including *Staphylococcus aureus,* the present inventor(s) seek to provide an antibacterial protein which can specifically lyse *Staphylococci,* including *Staphylococcus aureus.* Furthermore, the present inventor(s) further seek to provide a pharmaceutical composition, which contains this antibacterial protein as an active ingredient and can be used to treat infections caused by *Staphylococci.* Furthermore, the present inventor(s) further seek to provide a method of effectively treating infections caused by *Staphylococci* using the pharmaceutical composition.

Therefore, one objective of the present disclosure is to provide an antibacterial protein S2200, which has an antibacterial activity to specifically lyse *Staphylococci,* including *Staphylococcus aureus,* and contains an amino acid sequence represented by SEQ ID NO: 1.

Another objective of the present disclosure is to provide a method of efficiently preparing an antibacterial protein S2200, which has an antibacterial activity to specifically lyse *Staphylococci,* including the *Staphylococcus aureus,* and contains an amino acid sequence represented by SEQ ID NO: 1. In this regard, *Escherichia coli* Endo-22 is provided as a production strain of the antibacterial protein S2200. The production strain *Escherichia coli* Endo-22 was deposited at the Biological Resource Center of the Korea Research Institute of Bioscience and Biotechnology (KRIBB) on July 12, 2022 (Accession No. KCTC15031BP).

A further objective of the present disclosure is to provide a pharmaceutical composition for treating infections caused by *Staphylococci,* containing the antibacterial protein S2200 capable of specifically lysing *Staphylococci,* including *Staphylococcus aureus,* as an active ingredient.

In addition, a yet further objective of the present disclosure is to provide a method of treating infections caused by *Staphylococci,* including *Staphylococcus aureus,* by using the pharmaceutical composition containing the antibacterial protein S2200 as an active ingredient.

### Technical Solution

To achieve the objectives, the inventor(s) of the present disclosure utilized information on various antibacterial proteins known to have antibacterial activity against various bacterial species, and utilized their knowledge and expertise to the fullest to prepare various antibacterial protein candidates in the form of recombinant proteins and examined lytic activity of the antibacterial protein candidates against *Staphylococci,* including *Staphylococcus aureus.* Thereby, the inventor(s) selected an antibacterial protein with excellent lytic activity and developed a method of efficiently preparing the antibacterial protein, and lastly developed a pharmaceutical composition that can be used for treating infections caused by *Staphylococci,* including *Staphylococcus aureus,* by using the antibacterial protein as an active ingredient. As a result, the inventor(s) completed the present disclosure.

Therefore, according to one aspect of the present disclosure, the present disclosure provides an amino acid sequence of an antibacterial protein S2200, which may specifically lyse *Staphylococci,* including *Staphylococcus aureus.* Specifically, the amino acid sequence of the antibacterial protein S2200 corresponds to an amino acid sequence represented by SEQ ID NO: 1. The antibacterial protein S2200, which may specifically lyse *Staphylococci,* including *Staphylococcus aureus,* is made of 345 amino acid residues and has a molecular weight of approximately 38.0 kDa.

It is obvious that the amino acid sequence represented by SEQ ID NO: 1 may be partially modified by those skilled in the art using known techniques. The modifications include partial substitution of the amino acid sequence, partial addition of the amino acid sequence, and partial deletion of the amino acid sequence. However, it is most preferable to apply the amino acid sequence represented by SEQ ID NO: 1 disclosed in the present disclosure.

Additionally, the present disclosure provides a strain *Escherichia coli* Endo-22, which can be used to produce the antibacterial protein S2200 containing the amino acid sequence represented by SEQ ID NO: 1. The strain *Escherichia coli* Endo-22 is a strain to produce S2200, constructed by the present inventor(s) by transforming *E. coli* with a plasmid containing a nucleic acid sequence represented by SEQ ID NO: 2 (5,178 bp).

According to another aspect of the present disclosure, the present disclosure provides a pharmaceutical composition containing the antibacterial protein S2200 as an active ingredient for being effectively used for treating infections caused by *Staphylococci,* including *Staphylococcus aureus,* in which the antibacterial protein S2200 contains the amino acid sequence represented by SEQ ID NO: 1 and has specific lytic activity against *Staphylococci,* including *Staphylococcus aureus.*

According to the present disclosure, the antibacterial protein S2200, which is contained in the pharmaceutical composition of the present disclosure, contains the amino acid sequence represented by SEQ ID NO: 1 according to the present disclosure, and has specific lytic activity against *Staphylococci,* including *Staphylococcus aureus,* as described above, specifically lyses *Staphylococci,* including *Staphylococcus aureus.* Thus, the antibacterial protein S2200 is effective in treating various diseases caused by *Staphylococci,* including *Staphylococcus aureus.* Therefore, the pharmaceutical composition of the present disclosure may be used for treating diseases caused by *Staphylococci,* including *Staphylococcus aureus.* In addition, the pharmaceutical composition of the present disclosure is developed as an antibiotic, a disinfectant, a sterilizer, or a therapeutic agent to treat various diseases caused by *Staphylococci,* including *Staphylococcus aureus.*

Therefore, according to a further aspect of the present disclosure, the present disclosure provides a method of treating various diseases caused by *Staphylococci* by administering the antibacterial protein S2200 to individuals infected with *Staphylococci,* including *Staphylococcus aureus,* the antibacterial protein S2200 containing the amino acid sequence represented by SEQ ID NO: 1 and being specific for *Staphylococci,* including *Staphylococcus aureus.*

The term "disease caused by *Staphylococci",* as used herein, refers to a disease caused by staphylococcal infections. The disease refers to skin diseases, bacteremia, sepsis, endocarditis, or ulcers, but is not limited thereto.

It does not matter, in this specification, whether *Staphylococci,* including *Staphylococcus aureus* is sensitive to conventional antibiotics or resistant to conventional antibiotics. In other words, it does not matter whether *Staphylococci* acquires resistance to conventional antibiotics.

The term "treatment" or "treatment", as used herein, refers to all actions which suppress infections caused by *Staphylococci,* including *Staphylococcus aureus,* and alleviate pathological conditions of diseases caused by *Staphylococci,* including *Staphylococcus aureus.*

To increase efficiency for this purpose, antibacterial substances, which can provide antibacterial activity against other bacterial species, can be added to the pharmaceutical composition of the present disclosure.

A pharmaceutically acceptable carrier included in the pharmaceutical composition of the present disclosure is one commonly used in preparation. The pharmaceutically acceptable carrier may include any one selected from the group consisting of lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil, but is not limited thereto. In addition to the ingredients, the pharmaceutical composition of the present disclosure may further include any one selected from the group consisting of lubricants, wetting agents, sweeteners, flavoring agents, emulsifiers, suspending agents, and preservatives.

The pharmaceutical composition of the present disclosure may be administered through oral administration or parenteral administration. In the case of parenteral administration, the pharmaceutical composition may be administered using intravenous administration, intraperitoneal administration, intramuscular administration, subcutaneous administration, or local administration. In addition, application or spraying to the affected area may also be used, but the administration method is not limited thereto.

The pharmaceutical composition of the present disclosure may be formulated using the pharmaceutically acceptable carrier and/or an excipient by a method which can be easily performed by those skilled in the art to which the present disclosure pertains. Thereby, the pharmaceutical composition of the present disclosure may be prepared in unit dosage form or may be prepared by placing the pharmaceutical composition in a multi-dose container. In this case, the formulation of the pharmaceutical composition may be in the form of a solution, suspension, or emulsion in an oil or aqueous medium or may be in the form of an extract, a powder, a granule, a tablet, or a capsule. In the formulation, a dispersant or stabilizer may be further included.

In addition, suitable application, spraying, and dosage of the pharmaceutical composition vary depending on factors such as formulation method, administration method, age, weight, sex, severity of disease symptoms, food, administration time, administration route, excretion rate, and reaction sensitivity. Usually, a skilled doctor or veterinarian can easily determine and prescribe an effective dosage for the desired treatment.

The pharmaceutical composition of the present disclosure may be developed as an antibiotic, a disinfectant, a sterilizer, and a therapeutic agent.

### Advantageous Effects

A method of treating infections caused by *Staphylococci,* including *Staphylococcus aureus,* by using a pharmaceutical composition containing an antibacterial protein S2200 as an active ingredient, in which the antibacterial protein S2200 contains an amino acid sequence represented by SEQ ID NO: 1 according to the present disclosure, can also be effective against *Staphylococci* which have acquired resistance to conventional antibiotics or antibacterial substances. On the other hand, the antibacterial protein S2200 of the present disclosure specific for *Staphylococci,* including *Staphylococcus aureus,* does not affect normal flora other than *Staphylococcus aureus,* so it can minimize side effects resulting from the use of the pharmaceutical composition containing the antibacterial protein S2200 as an active ingredient. Meanwhile, conventional antibiotics have the disadvantages of harming beneficial bacteria and causing various side effects.

### Description of Drawings

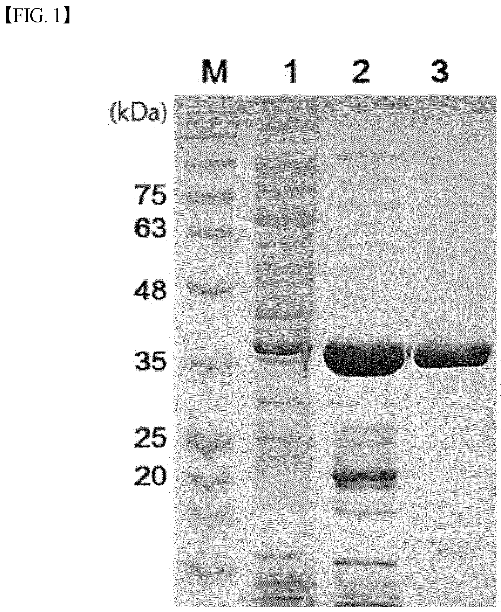

FIG. 1 shows an electrophoresis photograph showing the results of preparing an antibacterial protein S2200 specific for *Staphylococci,* including *Staphylococcus aureus* in the form of a recombinant protein, the antibacterial protein S2200 containing an amino acid sequence represented by SEQ ID NO: 1, in which lane M represents a protein size marker, lane 1 represents a cell lysate, lane 2 represents a chromatography flow-through during the first purification, and lane 3 represents a chromatography flow-through during the second purification.

### Best Mode

Hereinafter, the present disclosure will be described in more detail based on examples, but these examples are only illustrative of the present disclosure, and the scope of the present disclosure is not limited to these examples.

### Example 1: Preparation of Antibacterial Protein S2200 Specific for Staphylococci, Including Staphylococcus aureus

Preparation of an antibacterial protein S2200 specific for *Staphylococci,* including *Staphylococcus aureus,* will be described below. In this example, *Escherichia coli* Endo-22 was used as a production strain, constructed by the present inventor(s) by transforming *E. coli* with a plasmid containing a nucleic acid sequence represented by SEQ ID NO. 2.

20 µL of *Escherichia coli* Endo-22 was inoculated into 20 mL of LB medium (Tryptone 10 g/L, yeast extract 5 g/L, and NaCl 10 g/L) supplemented with 50 µg/mL kanamycin. Afterward, the LB medium was cultured with shaking at a temperature of 37°C overnight. The next day, the overnight cultured medium was added at a volume ratio of 1/100 to 1 L of LB medium supplemented with 50 µg/mL kanamycin. Culture was performed at a temperature of 37°C with a stirring speed of 200 rpm. When the cell concentration reached 0.3 to 0.35 based on absorbance at a wavelength of 600 nm, the culture temperature was changed to 19°C. After about 30 minutes, when the cell concentration reached 0.5 based on absorbance at a wavelength of 600 nm, L-arabinose was added so that the final concentration was 0.2%. Thereby, expression of an antibacterial protein S2200 containing an amino acid sequence represented by SEQ ID NO: 1 was induced. Thereafter, an additional 16-hour culture was performed.

After completion of the culture, the cell culture broth was taken and centrifuged at a temperature of 4°C for 10 minutes at 6,000 rpm to recover the cell pellet. The recovered cell pellet was suspended in 40 mL of a buffer solution (50 mM Na₂HPO₄, 10 mM EDTA, 1 mM DTT, and pH 7.5). The cells of the cell suspension prepared in this way were disrupted using a conventional sonication. The sonication was performed by applying ultrasonic waves for 3 seconds to break the cells, stopping for 3 seconds. This process was repeated for a total of 15 minutes and in an ice bath.

After the cell disruption, the cell lysate was centrifuged at 13,000 rpm for 20 minutes at a temperature of 4°C, and the obtained supernatant was subjected to conventional cation-exchange chromatography and hydrophobic interaction chromatography purification processes.

The purification process is briefly explained as follows. In this example, 5 mL of HiTrap ^{™}SP FF (GE Healthcare) was used as a cation-exchange resin, and 5 mL of Toyopearl PPG-600M (Tosoh Bioscience) was used as a hydrophobic interaction resin. In a first purification process, cation-exchange resin chromatography was performed after pre-equilibrating a column with a buffer A (25 mM Na₂HPO₄, 10 mM EDTA, and pH 7.5). A sample was loaded onto the column. Thereafter, the column was washed at a flow rate of 5 mL/min under the following conditions: 1.5 column volumes (CV) of the buffer A, 30 CV of a buffer B (25 mM Na₂HPO₄, 10 mM EDTA, 50 mM NaCl, 0.5% Triton X-100, and pH 7.5), and 5 CV of a mixture of the buffer A to a buffer C (25 mM Na₂HPO₄, 10 mM EDTA, 0.7 M NaCl, and pH 7.5), in which the mixture was adjusted so that the buffer C constituted 31% of the total volume. After the washing, cation-exchange chromatography, which was the first purification process, was performed under the following condition that a concentration gradient from the buffer A to the buffer C was changed from 31% to 100% at a flow rate of 5 mL/min. Next, hydrophobic interaction chromatography, which was a second purification process, was performed using the obtained first purification eluate. The hydrophobic interaction chromatography was performed after pre-equilibrating a column with a buffer D (25 mM Na₂HPO₄, 1.5 M NaCl, pH 7.5). The sample obtained during the first purification process was added dropwise to the column using 4 M NaCl to have the same conductivity value as the buffer D. Next, the column was washed by flowing 10 CV of a buffer E (10 mM L-Histidine, 1.5 M NaCl, and pH 7.5) at a flow rate of 5 mL/min. After the washing, chromatography was performed under the following condition that a concentration gradient from the buffer E to a buffer F (10 mM L-Histidine, 1.0 M Urea, and pH 7.5) was changed from 0% to 100% at a flow rate of 5 mL/min. In this process, elution of the antibacterial protein S2200 containing the desired amino acid sequence represented by SEQ ID NO: 1 was achieved. FIG. 1 shows the results of electrophoresis analysis of the antibacterial protein S2200 purified using a chromatographic purification process.

Among the obtained purified fractions, those containing high concentrations of the antibacterial protein S2200 were collected. The collected fractions were dialyzed against a buffer solution (10 mM L-Histidine, and pH 6.0) to perform medium exchange. Through this, it was possible to secure the antibacterial protein S2200 solution with a purity of 90% or more.

Buffer exchange was performed for this concentrated S2200 solution with a buffer (10 mM L-Histidine, 5%(w/v) Sorbitol, 0.1%(w/v) poloxamer 188, and pH 6.0). Thereby, "an S2200 pharmaceutical composition" was prepared.

### Example 2: Investigation of Antibacterial Activity of Antibacterial Protein S2200

Antibacterial activity of the antibacterial protein S2200 specific for *Staphylococci,* including *Staphylococcus aureus,* and prepared according to Example 1 was examined. Details of test strains subject to antibacterial activity investigation are shown in Table 1.

**[Table 1]**

| Test Strains Subject to Antibacterial Activity Investigation | | |
|---|---|---|
| Species | Strain | Source |
| *Staphylococcus aureus* | ATCC 33591 | ATCC |
| | ATCC 35556 | |
| | MW2 | ATCC |
| | CCARM 3271 | CCARM |
| | CCARM 3547 | |
| *Staphylococcus epidermidis* | KCTC 3752 | KCTC |
| *Staphylococcus haemolyticus* | ATCC 29970 | ATCC |
| *Acinetobacter baumannii* | CCARM 12228 | CCARM |
| *Clostridioides difficile* | CCARM 0184 | CCARM |
| *Pseudomonas aeruginosa* | CCARM 2247 | CCARM |
| *Klebsiella pneumoniae* | CCARM 10303 | CCARM |
| *Escherichia coli* | CCARM 1G931 | CCARM |

| Test Strains Subject to Antibacterial Activity Investigation | | |
|---|---|---|
| ATCC: American Type Culture Collection; | | |
| CCARM: Culture Collection of Antimicrobial Resistance Microbes; | | |
| KCTC: Korean Collection for Type Cultures | | |

A turbidity reduction assay and MIC (Minimum Inhibitory Concentration) test were used to investigate antibacterial activity. The turbidity reduction assay was conducted as follows. Test strains were suspended in physiological saline so that absorbance of the prepared cell suspension was about 0.7 at a wavelength of 600 nm. Thereafter, 0.1 mL of antibacterial protein S2200 solution was added (final concentration: 2.5 µM) to 0.9 mL of this cell suspension. Then, the absorbance was measured at a wavelength of 600 nm for 30 minutes. For preparing a negative control, a buffer (10 mM L-Histidine, 5%(w/v) Sorbitol, 0.1%(w/v) poloxamer 188, and pH 6.0) without the antibacterial protein S2200 was used instead of the antibacterial protein S2200 solution. An MIC was determined by conducting a routine MIC test in accordance with CLSI guidelines.

As a result, the antibacterial protein S2200 showed lytic activity only against *Staphylococci,* including *Staphylococcus aureus,* meanwhile the antibacterial protein S2200 did not show lytic activity against other test strains. The results are presented in Table 2.

**[Table 2]**

| Antibacterial Activity Investigation Results | | | |
|---|---|---|---|
| Species | Strain | Susceptibility | MIC (µg/ml) |
| *Staphylococcus aureus* | ATCC 33591 | Susceptible | 1 |
| | ATCC 35556 | Susceptible | 1 |
| | MW2 | Susceptible | 2 |
| | CCARM 3271 | Susceptible | 1 |
| | CCARM 3547 | Susceptible | 0.5 |
| *Staphylococcus epidermidis* | KCTC 3752 | Susceptible | 1 |
| *Staphylococcus haemolyticus* | ATCC 29970 | Susceptible | 1 |
| *Acinetobacter baumannii* | CCARM 12228 | Insusceptible | - |
| *Clostridioides difficile* | CCARM 0184 | Insusceptible | - |
| *Pseudomonas aeruginosa* | CCARM 2247 | Insusceptible | - |
| *Klebsiella pneumoniae* | CCARM 10303 | Insusceptible | - |
| *Escherichia coli* | CCARM 1G931 | Insusceptible | - |

From these results, it was confirmed that the antibacterial activity of the antibacterial protein S2200 of the present disclosure was significantly specific for *Staphylococci,* including *Staphylococcus aureus.*

From the results, it was confirmed that the antibacterial protein S2200 specific for *Staphylococci,* including *Staphylococcus aureus* of the present disclosure could lyse *Staphylococci,* including *Staphylococcus aureus,* thereby ultimately contributing to killing *Staphylococci,* including *Staphylococcus aureus.* These antibacterial properties of the antibacterial protein S2200 showed that the pharmaceutical composition containing the antibacterial protein S2200 specific for *Staphylococci,* including *Staphylococcus aureus* could be used for the purpose of killing *Staphylococci,* including *Staphylococcus aureus* when infections caused by *Staphylococci* occurred. In addition, the antibacterial properties of the antibacterial protein S2200 showed that the pharmaceutical composition containing the antibacterial protein S2200 specific for *Staphylococci,* including *Staphylococcus aureus* could also be used in the same way as conventional antibiotics for the purpose of treating infections caused by *Staphylococci.*

### Example 3: Investigation of Properties of S2200, Antibacterial Protein Specific for Staphylococci, Including Staphylococcus aureus

Immunogenicity of the antibacterial protein S2200 was investigated. The immunogenicity investigation experiment was conducted in two steps. A first step was preparation of 5,6-carboxyfluorescein diacetate succinimidyl ester (CFSE)-labeled peripheral blood mononuclear cells (PBMCs). The second step was T-cell proliferation assay and fluorescence-activated cell sorting (FACS) analysis. The CFSE-labeled PBMC preparation was performed as shown in Table 3 below.

**[Table 3]**

| CFSE-labeled PBMC Preparation | | | | |
|---|---|---|---|---|
| Date | No. | Step | Details | Remarks |
| Day 1 | 1 | Preparation | - Cell line: PBMC (Zen-bio, cat. SER-PBMC-F) | |
| | | | - Media: RPMI 1640 (Gibco, cat. 11875-093) with 10% fetal bovine serum (Gibco, cat. 12484-020) | |
| | 2 | Cell culture | - Pre-warm the media at a temperature of 37°C for 1 hour | 2-Day culture |
| | | | - Thaw 1 vial of PBMC at a temperature of 37°C for 10 minutes | |
| | | | - Prepare 5 mL of pre-warmed media in a 50 mL conical tube and add thawed PBMC | |
| | | | - Recover the cells (2,500 rpm, 3 min) | |
| | | | - Remove a supernatant, add 20 mL of pre-warmed new media to a cell pellet for resuspension, then transfer to a cell culture T-flask (75T) and culture the cells for 2 days (37°C, 5% CO₂) | |
| Day 3 | 3 | Preparation | - Prepare working solution by adding 18 µL of DMSC to 1 vial of CFSE (Thermo, cat. C34570) so that a final concentration is 5 mM | |
| | 4 | Staining | - Transfer the cultured cells to a 50 mL conical tube and recover the cells (2,500 rpm, 3 min) | |
| | | | - After removing the supernatant, resuspend the cell pellet in 1 mL of PBS and measure the number of cells using a cell counter (Lunar) | |
| | | | - Add media so that the number of cells is 1×10⁶ cells/mL, then add 1 µL (final 1 µM) of CFSE per 1 mL of media | |
| | | | - Perform incubation after blocking light (37°C, 20 min) | |
| | | | - Add pre-warmed media at 5 times the volume (cell + media) and incubate the media under light-blocked conditions (37°C, 5 min) | |
| | | | - Recover cells (2,500 rpm, 3 min) | |
| | | | - Remove a supernatant, resuspend a cell pellet in 1 mL of pre-warmed new media, measure the number of cells using a cell counter, and then dilute a cell suspension to 1×10⁶ cells/mL | |
| | | | - Use the diluted cells in experiments after stabilizing the diluted cells in an incubator for more than 10 minutes | |

A T-cell proliferation assay and FACS (Fluorescence-activated Cell Sorting) analysis were performed as shown in Table 4 below.

**[Table 4]**

| T-cell Proliferation Assay and FACS Analysis | | | |
|---|---|---|---|
| Date | No. | Step | Details |
| Day 1 | 1 | Sample Preparation | <Positive Control Preparation> |
| | | | - Use Dynabead^{™}Human T-Activator CD3/CD28 as positive control |
| | | | - Vortex beads (30 sec), calculate the ratio of the beads to cells to be 1:1, and transfer the beads and cells to a new tube |
| | | | - Calculate an amount of the beads as 2.5 µL per well |
| | | | - Add PBS in the same amount as the beads and vortex a mixture of the beads and PBS (1 min) |
| | | | - Use a magnet to immobilize the beads and remove a supernatant, then add an equal volume of medium to the previously added PBS and resuspend the beads tc prepare |
| | | | <S2200 Sample Preparation> |
| | | | - Prepare S2200 samples by diluting the S2200 samples with PBS to final concentrations of 200 µg/mL and 140 µg/mL, respectively |
| | 2 | T-cell Proliferatio n Assay | - Dispense 500 µL of the cell suspension per well to achieve a final concentration of 5×10⁵ cells/mL in each well, using a 24-well plate |
| | | | - Add the samples prepared in Step 1 to each well |
| | | | ① Negative control: Add 2.5 µL of PBS per well |
| | | | ② Positive control: Add 2.5 µL of CD3/CD28 beads per well |
| | | | ③ S2200 sample #1: Add 2.5 µL of the S2200 dilution (200 µg/mL) per well → a final application concentration of 1 µg/mL, 26 nM |
| | | | ④ S2200 sample #2: Add 2.5 µL of the S2200 dilution (140 µg/mL) per well → a final application concentration of 700 ng/mL, 18 nM |
| | | | - After dispensing the samples, incubate the samples at a temperature of 37°C with 5% CO₂, and on day 1 and day 5, sample one well each and measure fluorescence of CFSE using FACS |
| Day 2 (Day 1 of FACS Analysis ) | 1 | FACS Measurements | - Sample 500 µL of the cells in the well and transfer the sampled cells to a 1.5 mL tube |
| | | | - Recover the cells (3,000 rpm, 3 min) |
| | | | Add 500 µL of PBS for resuspension after removing a supernatant |
| | | | - Recover the cells (3,000 rpm, 3 min) |
| | | | - Add 500 µL of PBS to resuspend and perform FACS measurements after removing a supernatant |
| Day 6 (Day 5 of FACS Analysis ) | 1 | FACS Measurements | - Sample 500 µL of the cells and transfer the sampled cells to a 1.5 mL tube |
| | | | - Recover the cells (3,000 rpm, 3 min) |
| | | | - Add 500 µL of PBS for resuspension after removing a supernatant |
| | | | - Recover the cells (3,000 rpm, 3 min) |
| | | | - Add 500 µL of PBS to resuspend and perform FACS measurements after removing a supernatant |

The results of the immunogenicity investigation are shown in Table 5.

**[Table 5]**

| Immunogenicity Investigation Results | | | | |
|---|---|---|---|---|
| | Daughter cell (%) | | | |
| | NC_PBS | PC_CD3/CD23 | S2200 (26 nM) | S2200 (18 nM) |
| Day 1 | 0.40 | 27.23 | 0.51 | 0.32 |
| Day 5 | 0.73 | 67.62 | 0.87 | 0.39 |

The results showed that the immunogenicity of the antibacterial protein S2200 was significantly low. A clinical impact of immune responses in patients may range from minimal to significant harm. Considering the impact on pharmacokinetics, pharmacodynamics, safety, or efficacy, it was believed that the low immunogenicity of the antibacterial protein S2200 might be a great advantage in its use as a pharmaceutical.

### Example 4: Investigation of Therapeutic Effects of Antibacterial Protein S2200 Specific for Staphylococci, Including Staphylococcus aureus, on Staphylococcus aureus Infections

Therapeutic effects of the antibacterial protein S2200 specific for *Staphylococci,* including *Staphylococcus aureus* on *Staphylococcus aureus* infections were investigated through an infection animal model test. Specifically, female ICR mice (5 weeks old) weighing 23 g ± 20% [SPF grade, free of specific pathogens] were used. A total of 20 mice were divided into two groups (10 per group), and *Staphylococcus aureus* MW2 (1×10⁸ CFU/mouse) was intravenously injected into the two groups of mice. A buffer solution (10 mM L-Histidine, 5%(w/v) Sorbitol, 0.1%(w/v) poloxamer 188, and pH 6.0) was administered intravenously to the animals in the control group at 30 minutes, 12 hours, and 24 hours after the bacterial infections. The pharmaceutical composition prepared in Example 1 was intravenously administered to the animals in the treatment group (dose: 25 mg/kg) at 30 minutes, 12 hours, and 24 hours after the bacterial infections. Dead subjects were investigated, and clinical signs were observed daily. An ability of the pharmaceutical composition of the present disclosure to serve to kill bacteria in blood was confirmed by performing a conventional colony counting method. Tests were conducted using blood collected 5 days after the bacterial infections. Looking at the clinical signs and observation results, the animals in the treatment group were normal throughout the entire study period. Meanwhile, the animals in the control group showed various clinical signs, such as erythema of lid margin and decreased activity, starting 2 days after the bacterial infections. As a result of the investigation of the dead animals, it was confirmed that the survival rate of the animals in the treatment group administered the pharmaceutical composition of the present disclosure was significantly improved compared to those in the control group (Table 6).

**[Table 6]**

| Treatment Effect on *Staphylococcus aureus* Infections | | | | | | | |
|---|---|---|---|---|---|---|---|
| Group | Number of Dead Animals | | | | | Number of Dead Animals/Number of Test Animals | Mortality rate (%) |
| | Days after Forced Infections with Bacteria | | | | | | |
| | 1 | 2 | 3 | 4 | 5 | | |
| Control | 0 | 3 | 2 | 1 | 0 | 6/10 | 60 |
| Treatment | 0 | 0 | 0 | 0 | 0 | 0/10 | 0 |

Additionally, the administration of the pharmaceutical composition of the present disclosure significantly reduced the number of bacteria in the blood. An average number of bacteria in the blood was 1 × 10⁶ CFU/mL or more in blood collected from the animals in the control group. Meanwhile, no bacteria were observed in blood collected from the animals in the treatment group.

From the results, it was confirmed that the pharmaceutical composition prepared according to the present disclosure was effective in treating infections caused by *Staphylococcus aureus.* Accordingly, it could be concluded that the pharmaceutical composition of the present disclosure could be efficiently used for treating infections caused by *Staphylococcus aureus.*

### Example 5: Investigation of Therapeutic Effect of Antibacterial Protein S2200 Specific for Staphylococci, Including Staphylococcus aureus, on Staphylococcal Infections

Therapeutic effects of the antibacterial protein S2200 specific for *Staphylococci,* including *Staphylococcus aureus* on staphylococcal infections were investigated through an infection animal model test. Specifically, female ICR mice (5 weeks old) weighing 22 g ± 20% [SPF grade, free of specific pathogens] were used. A total of 20 mice were divided into two groups (10 per group), and *Staphylococcus hemolyticus* CCARM 3733 (1×10⁸ CFU/mouse) was intravenously injected into the two groups of mice. A buffer solution (10 mM L-Histidine, 5%(w/v) Sorbitol, 0.1%(w/v) poloxamer 188, and pH 6.0) was administered intravenously to the animals in the control group at 30 minutes, 12 hours, and 24 hours after the bacterial infections. The pharmaceutical composition prepared in Example 1 was intravenously administered to the animals in the treatment group (dose: 25 mg/kg) at 30 minutes, 12 hours, and 24 hours after the bacterial infections. Dead subjects were investigated, and clinical signs were observed daily. An ability of the pharmaceutical composition of the present disclosure to serve to kill bacteria in blood was confirmed by performing a conventional colony counting method. Tests were conducted using blood collected 5 days after the bacterial infections. Looking at the clinical signs and observation results, the animals in the treatment group were normal throughout the entire study period. Meanwhile, the animals in the control group showed various clinical signs, such as erythema of lid margin and decreased activity, starting 2 days after the bacterial infections. As a result of the investigation of the dead animals, it was confirmed that the administration of the pharmaceutical composition of the present disclosure significantly improved the survival rate of the animals (Table 7).

**[Table 7]**

| Treatment Effect on *Staphylococcal* Infections | | | | | | | |
|---|---|---|---|---|---|---|---|
| Group | Number of Deaths | | | | | Number of Dead Animals/Number of Test Animals | Mortality rate (%) |
| | Days after Forced Infections with Bacteria | | | | | | |
| | 1 | 2 | 3 | 4 | 5 | | |
| Control | 0 | 2 | 1 | 1 | 0 | 4/10 | 40 |
| Treatment | 0 | 0 | 0 | 0 | 0 | 0/10 | 0 |

Additionally, the administration of the pharmaceutical composition of the present disclosure significantly reduced the number of bacteria in the blood. An average number of bacteria in the blood was 1 × 10⁵ CFU/mL or more in blood collected from the animals in the control group. Meanwhile, no bacteria were observed in blood collected from the animals in the treatment group.

From the results, it was confirmed that the pharmaceutical composition prepared according to the present disclosure was also effective in treating infections caused by *Staphylococci.* Accordingly, it could be concluded that the pharmaceutical composition of the present disclosure could also be efficiently used for treating infections caused by *Staphylococci.*

The specific parts of the present disclosure have been described in detail above. For those skilled in the art, these specific techniques are merely examples of preferred implementations. It is clear that the scope of the present disclosure is not limited by these examples. Accordingly, the actual scope of the present disclosure will be defined by the appended claims and their equivalents.
Depository Institution Name: KCTC
Accession Number: KCTC15031BP
Deposit Date: 20220712

BUDAPEST TREATY ON THE INTERNATIONAL RECOGNITION OF THE DEPOSIT OF
MICROORGANISMS FOR THE PURPOSE OF PATENT PROCEDURE
INTERNATIONAL FORM
RECEIPT IN THE CASE OF AN ORIGINAL DEPOSIT
Issued pursuant to Rule 7.1

To: iNtRON Biotechnology
Address: 137, Sagimakgol-ro, Jungwon-gu, Seongnam-si, Gyeonggi-do, Republic
of Korea

I. IDENTIFICATION OF THE MICROORGANISM
Identification reference given by
the DEPOSITOR:
Escherichia coli Endo-22 | Accession number given by the
INTERNATIONAL DEPOSITARY
AUTHORITY:
KCTC 15031BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism
identified under I above, which was received by it on July 12, 2022.
IV. RECEIPT OF REQUEST FOR CONVERSION
This microorganism identified under I above was received by the
International Depositary Authority on and a request to convert the
original deposit to a deposit under the Budapest Treaty was received
by it on.
V. INTERNATIONAL DEPOSITARY AUTHORITY
Name: Korean Collection for Type | Signature(s) of person(s) having

Cultures | the power to represent the
| International Depositary Authority
Address: 181, Ipsin-gil, Jeongeup- | or of authorized official(s):
si, Jeonbuk-do, Republic of Korea |
(56212) | Representative
|
| Date: July 12, 2022

## Claims

1. An antibacterial protein S2200 with an amino acid sequence represented by SEQ ID NO: 1, which has specific antibacterial activity against *Staphylococci,* including *Staphylococcus aureus.*

2. The antibacterial protein S2200 of claim 1, wherein the antibacterial protein S2200 is made of 345 amino acids and has a molecular weight of 38.0 kDa.

3. A pharmaceutical composition for treating infections caused by *Staphylococci,* comprising *Staphylococcus aureus,* the pharmaceutical composition comprising the antibacterial protein S2200 of claim 1 as an active ingredient.

4. The pharmaceutical composition of claim 3, wherein the pharmaceutical composition is used as an antibiotic, a disinfectant, a sterilizer, or a therapeutic agent.

5. A method of preparing the antibacterial protein S2200 of claim 1 by using a production strain *Escherichia coli* Endo-22 (accession number KCTC15031BP) constructed by transforming *E. coli* with a plasmid containing a nucleic acid sequence represented by SEQ ID NO: 2.
